# EUROPEAN PATENT APPLICATION

(11) **EP 4 582 537 A1**
(43) Date of publication of application: **09.07.2025**
(21) Application number: 23860097.7
(22) Date of filing: 21.08.2023
(51) Int. Cl.: C12N 5/10, C12N 1/15, C12N 1/19, C12N 1/21, C12N 15/31, C12N 15/52, C12N 15/63, C12P 7/64

(54) **PROTEIN, POLYNUCLEOTIDE, EXPRESSION VECTOR, TRANSFORMANT, PLASMALOGEN-CONTAINING COMPOSITION, AND METHOD FOR PRODUCING PLASMALOGEN**

(30) Priority: 02.09.2022 JP 2022139676
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO Takehiko, Fukuoka-shi, Fukuoka 813-0043 (JP); MAWATARI Shiro, Fukuoka-shi, Fukuoka 813-0016 (JP); DOI Katsumi, Fukuoka-shi, Fukuoka 819-0395 (JP); HONSHO Masanori, Fukuoka-shi, Fukuoka 819-0395 (JP)
(74) Representative: V.O.
(86) International application number: PCT/JP2023/029944
(87) International publication number: WO 2024/048334

(57) **Abstract**

A protein includes an amino acid sequence selected from the group consisting of the following (a), (b), (c), and (d): (a) an amino acid sequence of SEQ ID NO:1; (b) an amino acid sequence having a sequence identity of not less than 80% to the amino acid sequence of SEQ ID NO: 1; (c) an amino acid sequence of SEQ ID NO:2; and (d) an amino acid sequence having a sequence identity of not less than 80% to the amino acid sequence of SEQ ID NO:2, the protein having plasmalogen synthesis activity in a bacterium that expresses the protein.

## Description

### Technical Field

The present disclosure relates to a protein, a polynucleotide, an expression vector, a transformant, a plasmalogen-containing composition, and a method of producing plasmalogen.

### Background Art

Plasmalogens are a type of phospholipids having antioxidant action, and are glycerophospholipids. Plasmalogens are present in all mammalian tissues, and account for about 18% of phospholipids in the human body. In particular, plasmalogens are known to be abundant in cranial nerves, cardiac muscle, skeletal muscle, leukocytes, and spermatozoa.

Since most plasmalogens are bound to polyunsaturated fatty acids such as docosahexaenoic acid and arachidonic acid, they are involved in, for example, the storage of polyunsaturated fatty acids, and the release of secondary messengers of intercellular signals such as prostaglandin and leukotriene produced from these polyunsaturated fatty acids. Plasmalogens are also involved in, for example, cell fusion and ion transport. Since vinyl ether bonds (alkenyl bonds) of plasmalogens are particularly sensitive to oxidative stress, plasmalogens also have antioxidant function for cells.

In addition, plasmalogens are known to have, for example, an action to promote neurogenesis, an action to suppress neuroinflammation due to lipopolysaccharides (LPS), and an action to suppress accumulation of amyloid β (Aβ) protein in the brain. For example, Patent Literature 1 discloses a drug against central nervous system inflammation, containing plasmalogen.

Plasmalogens are known to be decreased in cranial nerve diseases such as dementia, Parkinson's disease, depression, and schizophrenia; and also in diabetes, metabolic syndrome, ischemic heart disease, infections, and immune abnormalities. For example, Non Patent Literature 1 has reported that ethanolamine-type plasmalogen is highly significantly decreased in the frontal lobe and the hippocampus in the brain in Alzheimer's disease. Non Patent Literature 2 has reported that plasmalogens are decreased in sera of patients with Alzheimer's disease. Non Patent Literature 3 has reported that choline-type plasmalogen is decreased in a group of patients with ischemic heart disease compared to a normal control group.

An effect to prevent and ameliorate those diseases can be expected by externally supplementing the plasmalogen that has been decreased. Thus, for the prevention and treatment of the diseases, or for health maintenance, a demand for plasmalogens can be expected to increase in the future.

Plasmalogens are widely present in nonvertebrates and vertebrates, but have not been found in fungal and plant cells. In bacteria, synthesis of plasmalogens have been reported in several strictly anaerobic bacteria such as *Enterococcus faecalis* as disclosed in Non Patent Literature 4, and *Bifidobacterium longum* as disclosed in Patent Literature 2. However, no plasmalogen has been found in facultative anaerobic bacteria and aerobic bacteria (see Non Patent Literature 5).

Non Patent Literature 4 has identified two plasmalogen synthase genes (*plsA* and *plsR*)*,* and has reported that, when the expression of plasmalogen synthase of *Clostridium perfringens* was induced in aerobically grown *E. coli,* no plasmalogen was detected.

### Citation List

### Patent Literature

Patent Literature 1: International Publication No. WO 2012/039472
Patent Literature 2: International Publication No. WO 2021/024872

### Non Patent Literature

Non Patent Literature 1: Z. Guan et al, Decrease and Structural Modifications of Phosphatidylethanolamine Plasmalogen in the Brain with Alzheimer Disease, J Neuropathol Exp Neurol, 58 (7), 740-747, 1999
Non Patent Literature 2: D. B. Goodenowe et al, Peripheral ethanolamine plasmalogen deficiency: a logical causative factor in Alzheimer's disease and dementia, J Lipid Res, 48, 2485-2498, 2007
Non Patent Literature 3: Takeo Sanada and others, Serum Plasmalogens in Ischemic Heart Disease, The Journal of Japan Atherosclerosis Society, 11, 535-539, 1983
Non Patent Literature 4: David R Jackson et al, Plasmalogen Biosynthesis by Anaerobic Bacteria: Identification of a Two-Gene Operon Responsible for Plasmalogen Production in Clostridium perfringens, ACS Chemical Biology, 11, 12, 3421-3430, 2021
Non Patent Literature 5: Howard Goldfine, The appearance, disappearance and reappearance of plasmalogens in evolution, Progress in Lipid Research, 49, 4, 493-498, 2010

### Summary of Invention

### Technical Problem

If plasmalogen can be biosynthesized by a bacterium, a large amount of plasmalogen can be easily obtained by culturing the bacterium. However, in cases where plasmalogen is to be biosynthesized by a strictly anaerobic bacterium, the bacterium needs to be cultured under anaerobic conditions. Culturing under anaerobic conditions is laborious since it requires a device for which entrance of oxygen can be prevented, and since the maintenance of the anaerobic conditions becomes more difficult as the culture scale increases.

The present disclosure was carried out under such circumstances, and an objective of the present disclosure is to provide a protein, a polynucleotide, an expression vector, a transformant, a plasmalogen-containing composition, and a method of producing plasmalogen, for biosynthesizing plasmalogen by a bacterium capable of aerobic growth.

### Solution to Problem

The present inventors discovered that plasmalogen can be synthesized under aerobic conditions by introducing a plasmalogen synthase gene (*plsA*) derived from an anaerobic bacterium to an aerobic bacterium, thereby completing the present disclosure.

A protein according to a first aspect of the present disclosure is a protein, including:
an amino acid sequence selected from a group consisting of following (a), (b), (c), and (d):
   (a) an amino acid sequence of SEQ ID NO: 1;
   (b) an amino acid sequence having a sequence identity of not less than 80% to the amino acid sequence of SEQ ID NO: 1;
   (c) an amino acid sequence of SEQ ID NO:2; and
   (d) an amino acid sequence having a sequence identity of not less than 80% to the amino acid sequence of SEQ ID NO:2,
the protein having plasmalogen synthesis activity in a bacterium that expresses the protein.

A polynucleotide according to a second aspect of the present disclosure is a polynucleotide, including:
a base sequence encoding the protein according to the first aspect.

An expression vector according to a third aspect of the present disclosure is an expression vector, including:
the polynucleotide according to the second aspect.

A transformant according to a fourth aspect of the present disclosure is a transformant, including:
the expression vector according to the third aspect.

A plasmalogen-containing composition according to a fifth aspect of the present disclosure is a plasmalogen-containing composition, including:
the transformant according to the fourth aspect or a processed product thereof.

A method of producing plasmalogen according to a sixth aspect of the present disclosure is a method of producing plasmalogen, the method including:
culturing the transformant according to the fourth aspect under aerobic conditions; and
obtaining plasmalogen from the transformant.

### Advantageous Effects of Invention

According to the present disclosure, plasmalogen can be biosynthesized by a bacterium capable of aerobic growth.

### Brief Description of Drawings

FIG. 1 is a diagram illustrating the structure of *PlsA* derived from the *Enterococcus faecalis* K4 strain;
FIG. 2A is a diagram illustrating the CBB staining image in Example 1;
FIG. 2B is a diagram illustrating the Western blotting image in Example 1;
FIG. 3A is a diagram illustrating a chromatogram of the sample derived from the control *E. coli* (BL21) in Example 2;
FIG. 3B is a diagram illustrating a chromatogram of the sample derived from the transformed *E. coli* obtained in Example 1;
FIG. 4A is a diagram illustrating a chromatogram of the sample before the treatment with phospholipase A1 (PLA1) in Example 3;
FIG. 4B is a diagram illustrating a chromatogram of the sample after the treatment with PLA1 in Example 3;
FIG. 4C is a diagram illustrating a chromatogram of the sample treated with hydrochloric acid (HCl) after the treatment with PLA1 in Example 3;
FIG. 5A is a diagram illustrating the Western blotting image in Example 4;
FIG. 5B is a diagram illustrating the relative pAMPK/AMPK activity in Example 4;
FIG. 6 is a diagram illustrating the cells stained with Oil Red O in Example 5;
FIG. 7 is a diagram illustrating the Western blotting image in Example 6; and
FIG. 8 is a diagram illustrating a chromatogram for total lipids and aldehyde in Example 7.

### Description of Embodiments

Embodiments according to the present disclosure are described below with reference to drawings. The present disclosure is not limited by the following embodiments and drawings. In the following embodiments, expressions such as "have", "include", or "contain" also encompass the meaning of "consist of" or "be constituted by".

### Embodiment 1

The protein according to the present embodiment includes the amino acid sequence of SEQ ID NO:1 or SEQ ID NO:2, or an amino acid sequence having a sequence identity of not less than 80% to the amino acid sequence of SEQ ID NO: 1 or SEQ ID NO:2. The protein has plasmalogen synthesis activity that mediates biosynthesis of plasmalogen in a bacterium that expresses the protein, preferably in a bacterium that has been aerobically grown. The protein having the amino acid sequence of SEQ ID NO: 1 was identified in the *Enterococcus faecalis* K4 strain. The protein having the amino acid sequence of SEQ ID NO: 2 was identified in the *Bifidobacterium longum suis* subspecies.

When two amino acid sequences are aligned to achieve the highest degree of matching therebetween, the ratio of the number of sites where an identical amino acid is present in both amino acid sequences to the number of amino acids in the full length is called sequence identity. The ratio of the number of sites where an identical amino acid is present and the number of sites where similar amino acids are present to the number of amino acids in the full length is called sequence similarity.

A "similar amino acid" is another amino acid having a physicochemically similar side chain (conservative amino acid substitution). Amino acids having physicochemically similar side chains are classified, for example, as follows.
Aliphatic side chains: glycine (G), alanine (A), valine (V), leucine (L) and isoleucine (I)
Aliphatic-hydroxyl side chains: serine (S) and threonine (T)
Amide-containing side chains: asparagine (N) and glutamine (Q)
Aromatic side chains: phenylalanine (F), tyrosine (Y), and tryptophan (W)
Basic side chains: lysine (K), arginine (R), and histidine (H)
Acidic side chains: aspartic acid (D) and glutamic acid (E)
Sulfur-containing side chains: cysteine (C) and methionine (M)

The similar amino acids may also be amino acids having, to each other, a score higher than a predetermined threshold based on a matrix that defines similarity, such as PAM250 used in sequence analysis tools such as Clustal W. In cases where PAM250 is used, amino acids having high similarity to each other are, for example, amino acids having a score higher than 0.5 between the amino acids, and are amino acids classified into the same group in the following groups 1 to 9.
- Group 1: S, T, and A
- Group 2: N, E, Q, and K
- Group 3: N, H, Q, and K
- Group 4: N, D, E, and Q
- Group 5: Q, H, R, and K
- Group 6: M, I, L, and V
- Group 7: M, I, L, and F
- Group 8: H and Y
- Group 9: F, Y, and W

As the matrix defines the similarity, a known matrix may be employed. For example, BLOSUM or the like may be employed instead of PAM.

The "sequence identity of not less than 80%" regarding an amino acid sequence means a sequence identity of at least 80%. The amino acid sequence of the protein may have a sequence identity of not less than 80%, preferably not less than 85%, more preferably not less than 90%, still more preferably not less than 95%, still more preferably not less than 98%, especially preferably not less than 99% to the amino acid sequence of SEQ ID NO:1 as long as the protein has plasmalogen synthesis activity.

In cases where the protein according to the present embodiment includes an amino acid sequence having a sequence identity of not less than 80% to the amino acid sequence of SEQ ID NO: 1 or 2, the protein may contain an amino acid sequence for the improvement of the translation efficiency, and also an amino acid sequence for use in the purification of the protein, an amino acid sequence for the improvement of the expression efficiency such as chaperone, or the like as long as the protein has the plasmalogen synthesis activity. Examples of the amino acid sequence for use in the purification of the protein include a histidine tag, a SUMO tag, glutathione-S-transferase, and maltose-binding protein. The amino acid sequence for the improvement of the translation efficiency, the amino acid sequence for use in the purification, and the amino acid sequence for the improvement of the expression efficiency are added to at least one of the N-terminus or the C-terminus. The amino acids added to the N-terminus or the C-terminus are, for example, several amino acids, preferably 1 to 9 amino acids.

A method of producing the protein according to the present embodiment is described below. The protein can be chemically synthesized according to its amino acid sequence by a known method such as the solid-phase method or the liquid-phase method. The protein is preferably produced by a method using genetic engineering and molecular biology as follows.

First, the base sequence of the polynucleotide encoding the protein is determined. The base sequence is determined based on codons corresponding to the amino acids. Based on the correspondence relationship between amino acids and codons, one amino acid sequence can be assigned with a large number of base sequences each of which encodes the amino acid sequence. A computer may be used to determine the base sequence. Taking into account the codon usage of the host used to express the gene encoding the amino acid sequence, a suitable base sequence encoding the protein can be determined. The polynucleotides can be synthesized by a known method according to the base sequence. Alternatively, the polynucleotide may be obtained as an amplification product that is obtained by polymerase chain reaction (PCR) using, as a template, cDNA of the *Enterococcus faecalis* K4 strain or the *Bifidobacterium longum suis* subspecies.

Subsequently, an expression vector containing the polynucleotide encoding the protein is prepared. The expression vector is not limited as long as it can express the polynucleotide. The expression vector preferably contains a promoter sequence to which an RNA polymerase that allows the expression of the incorporated polynucleotide binds, an operator sequence, a ribosome binding site (RBS), and the like. In the expression vector, the polynucleotides may be placed under the control of the promoter sequence. As the promoter sequence, a known promoter sequence such as the T7 promoter can be used. When necessary, restriction enzyme sites for cloning may be added to the 5'-end and the 3'-end of the polynucleotide encoding the protein, and a base sequence encoding a histidine tag, a SUMO tag, or the like may be added to the 5'-end of the polynucleotide encoding the protein. In cases where a restriction enzyme is used, the base sequence of the restriction enzyme site included in the polynucleotide encoding the protein may be synonymously substituted.

By introducing the expression vector, for example, into a host capable of expressing an RNA polymerase that binds to the above-described promoter sequence in the presence of an expression inducer, the protein can be expressed in the host. The host is not limited, and is preferably a prokaryotic cell capable of aerobic growth. Specific examples of the host include *E. coli, Bacillus subtilis,* yeast, filamentous fungi, plant cells, and animal cells. From the viewpoint of efficient expression of the polynucleotide, E. *coli* is preferred.

The E. *coli* to be used as the host is not limited, and may be a known E. *coli.* The E. *coli* is preferably capable of inducing expression of the protein encoded by the polynucleotide in the presence of an expression inducer. Examples of the expression inducer include IPTG, rhamnose, and their combination. Examples of such E. *coli* include the BL21 (DE3) strain, which is a lysogenic bacterium of the bacteriophage λDE3, which has an RNA polymerase gene that expresses the protein in the presence of an expression inducer. The expression vector is preferably capable of inducing the expression of the polynucleotide by an RNA polymerase expressed in the presence of an expression inducer. Examples of the expression vector include pET vectors (such as pET-21a(+) and pET-24a(+)) and the pGEX vector.

Subsequently, the expression vector prepared as described above is introduced into a host, to prepare a transformant. Examples of the method of introducing the expression vector into the host include, but are not limited to, methods using competent cells prepared by the calcium chloride method or the rubidium chloride method; the electroporation method; and the protoplast method.

In cases where the expression vector described above is pET-21a(+), the E. *coli* strain BL21 (DE3) is preferably used as the transformant. Colonies of the transformant can be investigated by direct PCR or by determination of the base sequence of the DNA, to obtain a desired transformant.

In cases where the host colonies cannot be obtained, the expression vector may be an expression vector containing a promoter sequence to which an RNA polymerase that allows the expression of the protein in the presence of the expression inducer binds, and also containing a gene encoding the RNA polymerase, and a gene encoding lysozyme that inhibits the RNA polymerase when it is expressed in the absence of the expression inducer. The gene encoding the RNA polymerase and the gene encoding the lysozyme do not necessarily need to be included in the expression vector, and may be contained in the host to be transformed. In this case, the expression vector may be introduced into a host that contains the gene encoding the RNA polymerase expressed in the presence of the expression inducer and the gene encoding the lysozyme that inhibits the RNA polymerase when it is expressed in the absence of the expression inducer. Examples of such a host include a host in which a plasmid containing a gene encoding the lysozyme is introduced, such as the BL21 (DE3) pLysS strain. Thus, the transformant contains: the expression vector containing the polynucleotide encoding the protein, and the promoter sequence to which the RNA polymerase that allows the expression of the protein encoded by the polynucleotide binds; the gene encoding the RNA polymerase expressed in the presence of the expression inducer; and the gene encoding the lysozyme that inhibits the RNA polymerase when it is expressed in the absence of the expression inducer.

The transformant prepared is preferably cultured under aerobic conditions to synthesize plasmalogen. The aerobic conditions mean that the atmosphere contains oxygen. The aerobic conditions also include microaerobic conditions. In particular, the aerobic conditions mean performing culture without using reagents or equipment for the purpose of use in the culture of microorganisms under anaerobic conditions.

The method of culturing the transformant may be a known culture method suitable for the transformant. In cases where *E. coli* is used as the transformant, it may be cultured, for example, using LB agar medium or LB liquid medium.

Plasmalogens belong to a subclass specific to glycerophospholipids characterized by the presence of a vinyl ether bond at the sn-1 position, and an ester bond at the sn-2 position, of the glycerol backbone. The plasmalogen in the present embodiment is not limited as long as it is a glycerophospholipid that is generally classified as plasmalogen. Examples of the plasmalogen include ethanolamine plasmalogen (Pls), choline plasmalogen (PlsCho), inositol plasmalogen, and serine plasmalogen. Pls has a structure in which -CH₂CH₂2NH₂ is bound to the oxygen atom that is bound to the phosphorus at the sn-3 position of the glycerol backbone. PlsCho has a structure in which -CH₂CH₂N(CH₃)₃ is bound to the oxygen atom that is bound to the phosphorus at the sn-3 position of the glycerol backbone. In the plasmalogen synthesized by the transformant according to the present embodiment, the carbon chain bound to the oxygen atom at the sn-1 position has a variety of numbers of carbon atoms. Examples of the plasmalogen include not only mammalian plasmalogens, but also plasmalogens each having the carbon atoms in a number not found in mammals.

The transformant according to the present embodiment is capable of synthesizing plasmalogen even under aerobic conditions as shown in Examples below. Therefore, plasmalogen can be simply obtained without the need to maintain the culture atmosphere under anaerobic conditions.

### Embodiment 2

The plasmalogen-containing composition according to the present embodiment contains the transformant according to Embodiment 1 or a processed product thereof. In cases where the host of the transformant is a bacterium, bacterial cells can be obtained as a precipitate by centrifugation of the culture liquid after the culture.

The processed product of the bacterial cells are bacterial cells obtained by subjecting the above bacterial cells to a certain treatment, or a composition derived from the above bacterial cells or containing part of the above bacterial cells. Examples of such a processed product include freeze-dried bacterial cells, bacterial cells dried with acetone, extracts obtained by subjecting bacterial cells to solvent extraction treatment, and homogenates obtained by homogenization of bacterial cells or dried bacterial cells by sonication or the like. Examples of solvents that can be used for the solvent extraction include water, organic solvents, and mixtures thereof. Examples of the organic solvents include ether, chloroform, benzene, hexane, methanol, ethanol, isopropanol, and mixtures thereof. The extract obtained by the extraction using an extraction solvent can be used as it is in the form of a liquid, or can be used in the form of a liquid, gel, or paste after concentration or dilution. Further, the extract can be dried for use as a dried product. The drying can be performed by a known method such as spray drying, freeze drying, drying under reduced pressure, or fluidized drying.

The plasmalogen-containing composition according to the present embodiment contains plasmalogen synthesized by the transformant. Thus, the plasmalogen-containing composition is useful for externally supplementing plasmalogen in a living body. For example, the plasmalogen-containing composition is used for the treatment or prevention of a disease in which plasmalogen is decreased. Examples of the disease include inflammatory diseases, central nervous system inflammatory diseases, dementia, Parkinson's disease, depression, schizophrenia, diabetes, metabolic syndrome, ischemic heart disease, infections, and immune abnormalities.

In another embodiment, the plasmalogen-containing composition is used as a therapeutic agent or prophylactic agent for the diseases exemplified above in which plasmalogen is decreased. In another embodiment, the plasmalogen-containing composition is used as a therapeutic agent or prophylactic agent for the diseases exemplified above in which plasmalogen is decreased. Another embodiment provides an oral composition for the treatment of a disease in which plasmalogen is decreased, the composition containing a bacterial cell of the transformant according to Embodiment 1 or a processed product thereof. Specific examples of such an oral composition include supplements, food compositions, foods and beverages, functional foods, and food additives.

Another embodiment provides a method of producing plasmalogen using the transformant according to Embodiment 1. The method of producing plasmalogen includes a culture step and an acquisition step. In the culture step, the transformant is cultured under aerobic conditions as described above. In the acquisition step, plasmalogen is obtained from the transformant. In the acquisition step, plasmalogen can be obtained by treating bacterial cells by, for example, solvent extraction, or homogenization by sonication or the like as described above. The plasmalogen obtained in the acquisition step may be purified by a known method, or the plasmalogen may be isolated. The purified plasmalogen may be concentrated.

### Example 1. Preparation of E. coli Transformed with Plasmalogen Synthase Gene Derived from Enterococcus faecalis K4 Strain

### [Cloning of K4 plsA]

Searching for the *plsA* gene derived from the facultative anaerobic lactic acid bacterium *E. faecalis* K4 strain (hereinafter also referred to as *"K4plsA"*) was performed by the following method.

Draft genome analysis was performed by 250PE (paired-end) using Hiseq (registered trademark) 2500 (manufactured by Illumina, Inc.), and De novo analysis by MinION (registered trademark) (manufactured by Oxford Nanopore Technologies plc.), to prepare a CDS list. By blast analysis using it, one gene (*K4plsA*) that was assumed to be hydroxyacyl-CoA dehydratase was found.

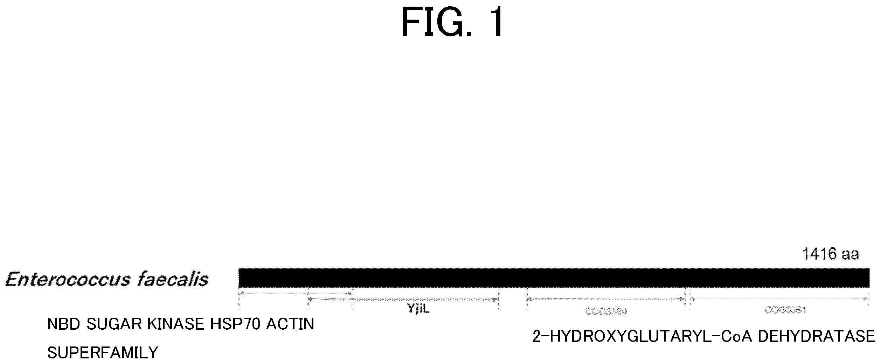

According to the above analysis, it is thought that the amino acid sequence of the plasmalogen synthase protein derived from the *E. faecalis* K4 strain (hereafter also referred to as "K4 PlsA protein") is the sequence of 1416 amino acids of SEQ ID NO: 1, and that, based on prediction by NCBI Conserved Domains (NCBI, https://www.ncbi.nlm.nih.gov/Structure/cdd/cdd.shtml), the amino acid sequence includes, in the order from the N-terminal side, an NBD sugar kinase HSP70 actin superfamily domain of 257 amino acids, a YjiL domain of 429 amino acids, and 2-hydroxyglutaryl-CoA dehydratase D components of 355 amino acids and 402 amino acids (FIG. 1). According to GENETYX-MAC (manufactured by GENETYX Corporation), the estimated molecular weight was 156,990, and the estimated isoelectric point was 5.77.

The *E. faecalis* K4 strain was cultured in BD Difco (trademark) Lactobacilli MRS Broth (manufactured by Becton-Dickinson, Japan) at 37°C overnight, to obtain genomic DNA. PCR was carried out using the obtained genomic DNA as a template, and using a forward primer having the base sequence of SEQ ID NO:3 and a reverse primer having the base sequence of SEQ ID NO:4, to amplify DNA of a gene that was assumed to be *plsA,* such that the initiation codon and the stop codon were excluded and such that a histidine tag and a SUMO tag were added to the 5'-end. The resulting PCR product was inserted into the expression vector pETite N-HIS SUMO Expression Vector (manufactured by LGC Biosearch Technologies), to obtain a recombinant plasmid (pETite N-HIS SUMO/plsA).

The resulting recombinant plasmid was introduced into *E. coli* (HI-Control 10G Chemically Competent Cells (manufactured by LGC Biosearch Technologies)), to obtain transformed *E. coli.* The resulting transformed *E. coli* was cultured at 37°C overnight in 100 mL of LB medium supplemented with 30 µg/mL kanamycin. The resulting culture liquid was centrifuged to collect bacterial cells. The plasmid was purified from the bacterial cells using the QIAprep Spin Miniprep Kit (manufactured by Qiagen). Using the purified plasmid, HI-Control BL21 (DE3) Chemically Competent Cells (manufactured by LGC Biosearch Technologies) were transformed.

### [Expression of K4 PlsA in Transformed E. coli]

The resulting transformed *E. coli* was cultured at 30°C overnight in LB medium supplemented with 30 µg/mL kanamycin. As a control, untransformed *E. coli* (BL21 strain) was cultured at 30°C overnight in LB medium not supplemented with kanamycin. To ZYM5052 medium (Studier, F. W. Protein Expr. Purif. 41, 207-234), 300 µL of each culture liquid was transferred, and culture was carried out at 30°C for 24 hours under anaerobic conditions or aerobic conditions. The culture liquid was centrifuged, and subjected to CBB staining, and Western blotting using an anti-histidine antibody.

### Results

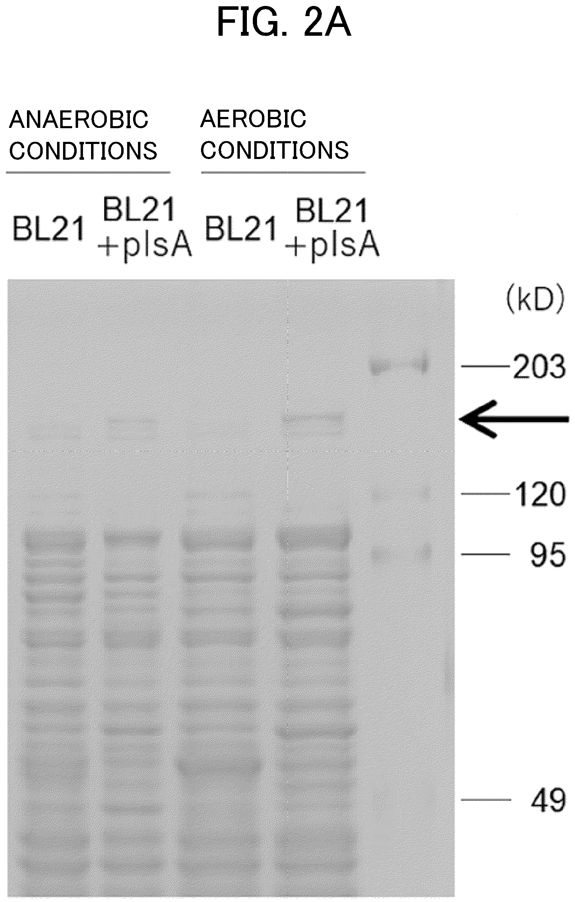

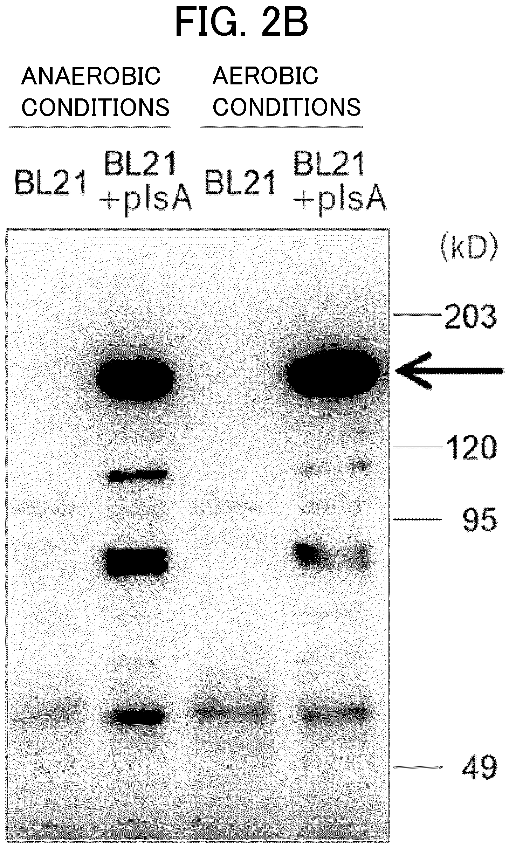

FIGS. 2A and 2B illustrate the results of the CBB staining and the Western blotting, respectively. "BL21" represents the untransformed *E. coli,* and "BL21+plsA" represents the transformed *E. coli* obtained in the present Example. As indicated by the arrow in FIG. 2B, expression of K4 PlsA protein was found in both the transformed E. *coli* cultured under the anaerobic conditions and the transformed *E. coli* cultured under the aerobic conditions.

### Example 2. Production of Plasmalogen in K4 plsA-Transformed E. coli [Extraction of Lipids from Bacterial Cells, and Detection of Phospholipids]

The transformed *E. coli* obtained in Example 1, and untransformed *E. coli* (BL21 strain) as a control were subjected to shake culture for 18 hours in the presence of air. The culture liquid after the culture was centrifuged to collect bacterial cells. Physiological saline was added to and mixed with the bacterial cells, and the resulting mixture was centrifuged, followed by removal of the supernatant, to wash the bacterial cells. Physiological saline was further added to the bacterial cells, to wash the cells again. After removing the supernatant, the bacterial cells were stored at -30°C until use.

After thawing the bacterial cells, the total weight of the cells was measured. After 1 mL of water was added to and mixed with the bacterial cells, 3.75 mL of chloroform/methanol (1:2, v/v) was added thereto, and the resulting mixture was mixed. The mixture was subjected to sonication for 10 minutes, and then left to stand at room temperature for 30 minutes. After adding 1.25 mL chloroform to the mixture, 1.25 mL water was added thereto. After performing centrifugation, the chloroform layer was transferred to a glass tube, and the remaining aqueous layer was subjected to re-extraction with 2 mL of chloroform. The resulting combined chloroform layer was dried under nitrogen gas, and resuspended in hexane/isopropanol (3:2, v/v) to a bacterial cell concentration of 120 mg/mL, followed by filtration through a 0.45-µm filter. After injecting 10 µL of the bacterial cell suspension into high-performance liquid chromatography (HPLC), detection was performed by an evaporative light-scattering detector (ELSD).

To provide a lipid standard sample, phosphatidylcholine (25 mg; 5 mg/mL in chloroform; A-29B, manufactured by SRL) was subjected to evaporation to dryness with nitrogen gas, and 1 mL of isopropanol/chloroform (2:1, v/v) was added thereto, followed by mixing the resulting mixture. The sample was dissolved by sonication, and stored as a lipid stock at -30°C. The lipid stock was serially diluted with HIP (hexane/isopropanol (3:2, v/v)), to prepare lipid standard solutions. After passing each lipid standard solution through a 0.45-µm filter, 10 µL of the resulting filtrate was used as a sample for HPLC.

The HPLC conditions were as follows.
Column: Lichrosphere DIOL (250 × 3 mm, 5 µm, manufactured by Merck)
Mobile phase A: Hexane/isopropanol/acetic acid (82:17:1) containing 0.08% triethylamine
Mobile phase B: Isopropanol/water/acetic acid (85:14:1) containing 0.08% triethylamine
Flow rate: 0.8 mL/minute
Column temperature: 50°C
Gradient:

| | | |
|---|---|---|
| Minute 0 | Mobile phase A 96% | Mobile phase B 4% |
| Minute 21 | Mobile phase A 63% | Mobile phase B 37% |
| Minute 25 | Mobile phase A 15% | Mobile phase B 85% |
| Minute 26 | Mobile phase A 15% | Mobile phase B 85% |
| Minute 29 | Mobile phase A 96% | Mobile phase B 4% |
| Minute 34 | Mobile phase A 96% | Mobile phase B 4% |

The ELSD conditions were as follows.
Evaporator temperature: 60°C
Nebulizer temperature: 30°C
Nitrogen gas flow rate: 1.00 SLM

Lipid standard solutions with different concentrations were also detected by HPLC-ELSD. A calibration curve was prepared based on the peak area at each concentration, and the amount of phospholipids was calculated from the area ratio of the peak corresponding to phospholipids obtained from the *E. coli* with a retention time of 10 to 20 minutes.

### [Change in Plasmalogen by Acid Hydrolysis Treatment]

Lipids were extracted from bacterial cells using chloroform/methanol, and dried with nitrogen gas as described above. To the lipids, 0.5 mL of 2,4-dinitrophenylhydrazine-hydrochloride solution (DNPH-HCl solution, manufactured by Tokyo Chemical Industry Co., Ltd.) was added, and the resulting mixture was mixed. The mixture was emulsified by sonication, and then left to stand at room temperature for 30 minutes to allow acid hydrolysis of the lipids. Thereafter, 0.5 mL of ultrapure water was added to and mixed with the resulting acid hydrolysate, and 1.9 mL of chloroform/methanol (1:2, v/v) was added to and mixed with the resulting mixture. The resulting mixture was left to stand at room temperature for 10 minutes, and then 0.625 mL of chloroform was added to and mixed with the resulting mixture. Subsequently, 0.625 mL of water was added to and mixed with the resulting mixture. After performing centrifugation at 3000 rpm at room temperature, the chloroform layer was transferred to a glass tube, and then dried under nitrogen gas. The dried lipids were resuspended in 100 µL of acetonitrile, and then passed through a 0.45-µm filter. As a sample for HPLC, 10 µL of the resulting filtrate was injected into HPLC, and detection was performed with ultraviolet (UV) at a wavelength of 356 nm.

The aldehyde standard solutions used as standards were prepared as follows. Tetradecanol (C₁₄H₂₈O, manufactured by Tokyo Chemical Industry Co., Ltd., T2696), hexadecanol (C₁₆H₃₂O, manufactured by Tokyo Chemical Industry Co., Ltd., H1296), heptadecanol (C₁₇H₃₄O, manufactured by Tokyo Chemical Industry Co., Ltd., H1295), and octadecanol (C₁₈H₃₆O, manufactured by Tokyo Chemical Industry Co., Ltd., O0368) were placed in separate spitz tubes, and acetonitrile was added thereto to achieve a concentration of 1 mg/mL. Each prepared solution at 1 mg/mL in an amount of 200 µL (100 µL, 200 µL, or 400 µL only in the case of C₁₄H₂₈O) was placed in a separate spitz tube, and evaporated to dryness with nitrogen gas. Thereafter, 0.5 mL of a DNPH-HCl solution was added to and mixed with the resulting sample. The sample was then emulsified by sonication, and left to stand at room temperature for 30 minutes. To the sample, 0.5 mL of ultrapure water and 1.9 mL of chloroform/methanol (1:2, v/v) were added, and the resulting mixture was mixed by vortexing. The mixture was then left to stand at room temperature for 10 minutes, and 0.625 mL of chloroform was added thereto, followed by mixing the resulting mixture by vortexing. To the sample, 0.625 mL of ultrapure water was added. The resulting mixture was mixed by vortexing. The sample was centrifuged (3000 rpm) at room temperature for 5 minutes. The lower layer was transferred to another spitz tube, and evaporated to dryness with nitrogen gas. The resulting sample was dissolved in 2 mL of acetonitrile, and then stored at -30°C as a stock aldehyde standard solution.

After taking 120 µL of the C₁₄H₂₈O stock aldehyde standard solution with each concentration, it was filtered through a 0.45-µm filter, and 10 µL of the resulting filtrate was injected into HPLC. In order to prepare a mixed aldehyde standard solution of the four kinds of aldehydes, 30 µL each of the four stock aldehyde standard solutions was taken and mixed together. The resulting mixed aldehyde standard solution was filtered through a 0.45-µm filter, and 10 µL of the resulting filtrate was injected into the HPLC.

The HPLC conditions were as follows.
Column: XBridge BEA C18 (3.0 × 150, 2.5 µm, manufactured by Waters)
Column temperature: 40°C
Mobile phase A: Acetonitrile
Mobile phase B: Water
Flow rate: 0.3 mL/minute
Column temperature: 40°C
Gradient:

| | | |
|---|---|---|
| Minute 0 | Mobile phase A 30% | Mobile phase B 70% |
| Minute 25 | Mobile phase A 100% | Mobile phase B 0% |
| Minute 40 | Mobile phase A 100% | Mobile phase B 0% |
| Minute 40.1 | Mobile phase A 30% | Mobile phase B 70% |
| Minute 50 | Mobile phase A 30% | Mobile phase B 70% |

For the aldehydes, a calculation was performed based on the area ratio in the same manner as for the phospholipids.

### Results

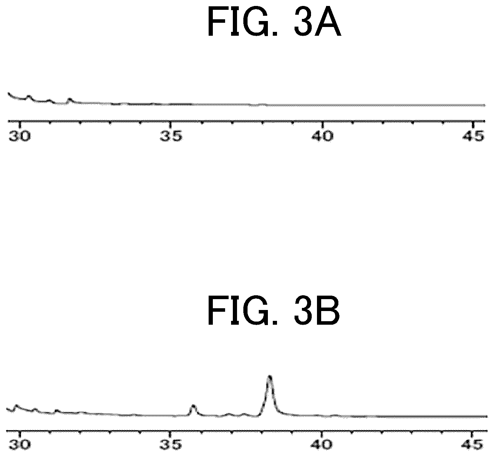

FIG. 3 illustrates chromatograms of the samples of the transformed *E*. *coli* according to Example 1 and the control *E*. *coli,* as detected by UV at a wavelength of 356 nm. FIGS. 3A and 3B illustrate the result on the control E. *coli* (BL21), and the result on the transformed *E*. *coli* according to Example 1, respectively. While no aldehyde was detected in the control *E*. *coli* sample, aldehyde was detected in the transformed *E*. *coli* sample. Since acid hydrolysis of plasmalogen generates aldehyde, the transformed *E. coli* was shown to produce plasmalogen when it is aerobically grown.

### Example 3. Changes in Plasmalogen by Phospholipase A1 (PLA1) Treatment and Hydrochloric Acid (HCl) Treatment

The transformed *E*. *coli* obtained in Example 1 was subjected to static culture under anaerobic conditions in ZYM5052 medium using AnaeroPack. Lipids were extracted from bacterial cells according to the procedure described in Example 2.

### [Change in Phospholipids by Hydrolysis Treatment of Total Lipids by Phospholipase A1 (PLA1)]

A certain amount of total lipids were hydrolyzed with PLA1. After preparing 0.5 mL of a PLA1 solution purchased from SIGMA with 0.5 mL of 0.1 M citrate buffer (pH 4.5), 1 mL of the resulting enzyme solution was added to the total lipid extract. The resulting mixture was emulsified in an ultrasonic bath, and the resulting suspension was incubated at 45°C for 60 minutes. Lipids were extracted with hexane/isopropanol, and the resulting combined hexane layer was dried under nitrogen gas. The lipids were mixed again with hexane/isopropanol (3:2, v/v), and the resulting mixture was injected into HPLC-ELSD. As the PLA1, PLA1 (Phospholipase A1) derived from *Aspergillus oryzae* (manufactured by SIGMA) was used. The minimum activity of the enzyme is 10 KLU/G liquid.

### [Change in Ether Phospholipid (Plasmalogen) by Acid Hydrolysis Treatment]

Subsequently, ether phospholipid (plasmalogen) was acid hydrolyzed using a 2,4-dinitrophenylhydrazine-hydrochloride solution (DNPH-HCl solution). After the PLA1 hydrolysis, 0.5 mL of the DNPH-HCl solution was added to the dried ether phospholipid. The resulting mixture was left to stand at room temperature for 30 minutes, and the hydrolyzed ether phospholipid was extracted by the chloroform-methanol method described above, followed by drying the chloroform layer under nitrogen gas. The dried lipids were mixed again with hexane/isopropanol (3:2, v/v), and a certain amount of the resulting mixture was injected into HPLC-ELSD.

### Results

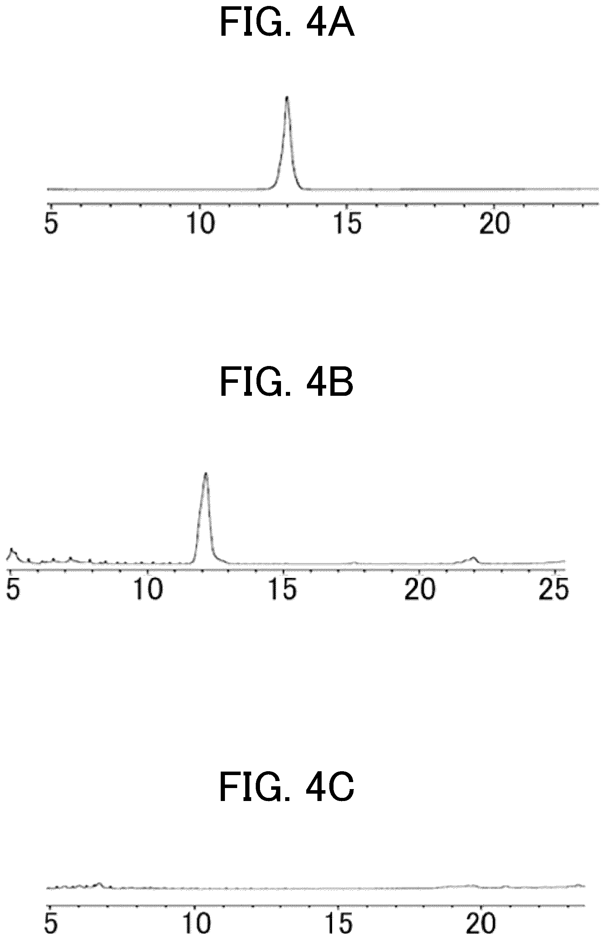

FIGS. 4A, 4B, and 4C are chromatograms of the samples for the total lipids before the PLA1 treatment, after the PLA1 treatment, and after the PLA1 treatment followed by the hydrochloric acid treatment, respectively. By the PLA1 treatment, phospholipids other than ether phospholipid and sphingomyelin are degraded, but plasmalogen remains. In cases where the hydrochloric acid treatment is carried out after the PLA1 treatment, plasmalogen is selectively degraded. It was thus shown, from these chromatograms, that plasmalogen is produced in the anaerobically grown transformed *E. coli.*

### Example 4. Phosphorylation of AMP-Activated Protein Kinase (AMPK) by Purified Plasmalogen

In OPTI-MEM (manufactured by Invitrogen), 0.5 µg of plasmalogen purified from the transformed *E*. *coli* of Example 1 was suspended, and the resulting suspension was added to a culture liquid (DMEM/10%FCS) of NIH-3T3. Cells were collected 3 hours later. Subsequently, Western blotting was carried out using an anti-pAMPK antibody (manufactured by Cell Signaling, catalog number 2531) and an anti-AMPK antibody (manufactured by Cell Signaling, catalog number 5831). In addition, AMPK and phosphorylated AMPK (pAMPK) proteins were quantified, and the relative pAMPK/AMPK value was determined.

### Results

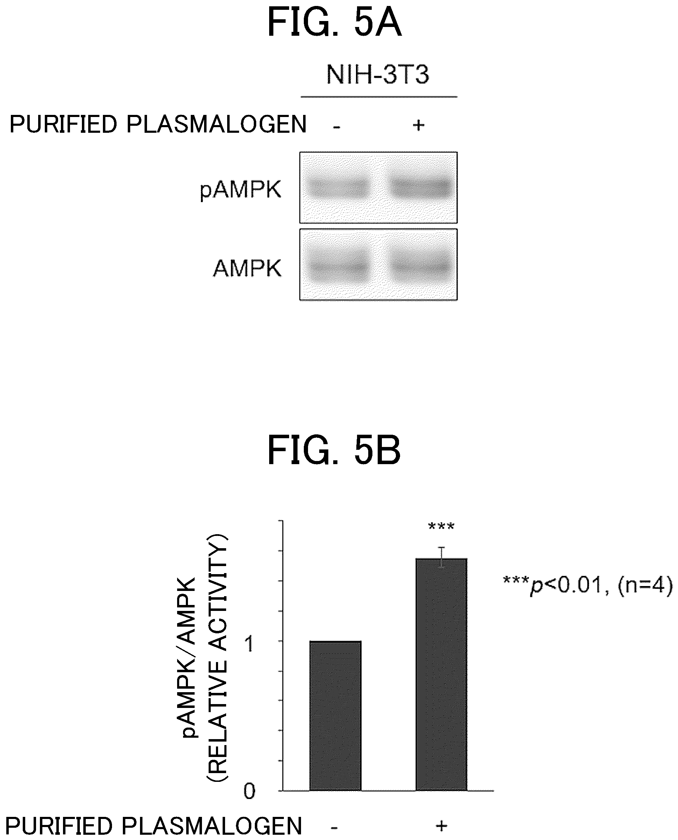

The result of the Western blotting is illustrated in FIG. 5A, and the relative pAMPK/AMPK activity is illustrated in FIG. 5B. In each panel, the left side corresponds to the control sample to which the purified plasmalogen was not added, and the right side corresponds to the sample to which the purified plasmalogen was added. The sample to which the purified plasmalogen was added showed a statistically significantly higher pAMPK/AMPK value (p<0.01). Thus, the plasmalogen synthesized by the transformed *E*. *coli* of Example 1 was shown to contribute to the phosphorylation of AMPK. AMPK is known to be activated by phosphorylation, to be involved in the glucose metabolism, lipid metabolism, and the like. Therefore, the plasmalogen was suggested to be effective for the prevention and treatment of various diseases related to the glucose metabolism or lipid metabolism, such as type 2 diabetes, dyslipidemia, and obesity.

### Example 5. Inhibition of Lipid Droplet Formation, by Purified Plasmalogen

Oleic acid (manufactured by Cayman Chemical) was preliminarily mixed with 4% defat-BSA solution. To DMEM/10%FCS culture liquid containing olein sign at a final concentration of 150 µM, 0.5 µg/mL of plasmalogen purified from the transformed *E. coli* of Example 1 was added. Mouse fetal fibroblasts (NIH-3T3 cells) were cultured therein for 18 hours. To provide a control, culture was performed with a culture liquid containing no plasmalogen. Cells were collected, washed, and then fixed with 4% paraformaldehyde solution. Subsequently, the cells were treated with 60% isopropanol solution, and stained with an OilRed O staining solution (prepared by 1.7-fold dilution of OilRed O (manufactured by Sigma) with water) for 15 minutes, followed by washing with 60% isopropanol, 30 seconds of staining with a hematoxylin solution (prepared by 10-fold dilution of hematoxylin (manufactured by Merk) with water), washing with water, embedding, and then observation.

### Results

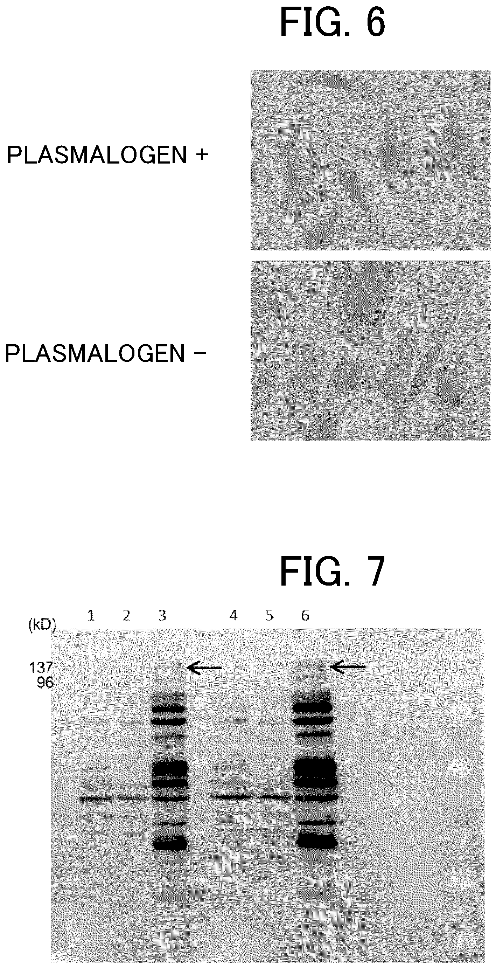

Photographs of the cells stained with OilRed O are illustrated in FIG. 6. While a large number of lipid droplets were found in the control cultured in the culture liquid containing no purified plasmalogen (plasmalogen -), lipid droplets were hardly found in the cells cultured in the culture liquid containing the purified plasmalogen (plasmalogen +). Thus, the plasmalogen synthesized by the transformed *E. coli* of Example 1 was shown to contribute to the suppression of fat formation or to the fat degradation, suggesting that the plasmalogen is effective for the prevention and treatment of dyslipidemia, obesity, and the like.

### Example 6. Preparation of Transformed E. coli with Artificial plsA Gene Derived from Bifidobacterium longum suis Subspecies

### [Cloning of suis plsA]

By the following method, artificial synthesis was carried out to prepare an artificial *plsA* gene derived from the *B. longum suis* subspecies (which may be hereinafter also referred to as *"suis plsA"*)*,* which is an obligate anaerobic bifidobacterium.

The region of 65,667 to 70,586 in the deposited genome sequence of the reference strain DSM20211 of the *B. longum suis* subspecies (*Bifidobacterium longum* subsp. *suis* DSM 20211 contig3, whole genome shotgun sequence:
NZ_JDUC01000003.1) was selected for the artificial synthesis of a base sequence optimized for the expression in *E. coli,* using GeneArt (registered trademark) GeneOptimizer (registered trademark) (manufactured by Thermo Fisher Scientific Inc.). The base sequence was then ligated into the cloning site of the pET100 Directional TOPO vector (manufactured by Thermo Fisher Scientific Inc.) together with CACC (single-stranded DNA).

It is thought that the amino acid sequence of the PlsA protein translated from this gene (hereafter also referred to as "suis PlsA protein") is the sequence of 1639 amino acids of SEQ ID NO:2, and that, based on prediction by NCBI Conserved Domains (NCBI, https://www.ncbi.nlm.nih.gov/Structure/cdd/cdd.shtml), the amino acid sequence includes, in the order from the N-terminal side, an NBD sugar kinase HSP70 actin superfamily domain of 257 amino acids, a YjiL domain of 429 amino acids, a COG3580 domain of 370 amino acids, and a 2-hydroxyglutaryl-CoA dehydratase D-component of 401 amino acids. According to GENETYX-MAC (manufactured by GENETYX Corporation), the estimated molecular weight was 178,715, and the estimated isoelectric point was 5.90.

The resulting recombinant plasmid was introduced into *E. coli* BL21 (DE3) competent cells (ChampionTM21; manufactured by SMOBIO Technology), to obtain transformed *E. coli.*

The resulting transformed *E. coli* was cultured at 37°C overnight in 10 mL of LB medium supplemented with 50 µg/mL ampicillin. Three milliliters of the resulting culture liquid was cultured at 37°C overnight in 100 mL of Overnight Express (registered trademark) Instant TB Medium (manufactured by Merck). The resulting culture liquid was centrifuged to collect bacterial cells. The bacterial cells were suspended in 20 mM Tris-HCl buffer (pH 7.0), and then sonicated to obtain an extract derived from the transformed *E. coli.* Further, the cell extract was centrifuged at 14,000 rpm for 15 minutes, to obtain a supernatant.

### [Expression of suis PlsA Protein by Transformed E. coli]

In order to confirm whether the resulting transformed *E. coli* expresses the desired suis PlsA protein under aerobic conditions, samples were prepared by performing culture by the following three kinds of methods.

Culture Method 1: The transformed *E. coli* was precultured in LB medium at 37°C at 150 rpm overnight with shaking. Thereafter, the resulting preculture was inoculated at 3% into 10 mL of fresh LB medium, and shake culture was performed at 37°C at 150 rpm overnight.

Culture Method 2: The transformed *E. coli* was precultured in LB medium at 37°C at 150 rpm overnight with shaking. Thereafter, the resulting preculture was inoculated at 3% into 10 mL of fresh LB medium, and shake culture was performed at 37°C at 150 rpm. At the time when OD660 reached 0.4, IPTG induction (0.5 mM) was carried out, and then shake culture was performed at 37°C at 150 rpm for 3 hours.

Culture Method 3: The transformed *E. coli* was precultured in LB medium at 37°C at 150 rpm overnight with shaking. Thereafter, the resulting preculture was inoculated at 3% into 10 mL of Overnight Express (registered trademark) Instant TB Medium (manufactured by Merck), and shake culture was performed at 37°C at 150 rpm overnight.

The resulting culture liquid was centrifuged to collect bacterial cells. The bacterial cells were suspended in 20 mM Tris-HCl buffer (pH 7.0), and then sonicated to obtain an extract derived from the transformed *E. coli.* Further, the cell extract was centrifuged at 14,000 rpm for 15 minutes, to obtain a supernatant. Each sample was dissolved in an electrophoresis buffer such that the bacterial cell weight per volume was constant. The same volume of each solution was subjected to electrophoresis, and then Western blotting was carried out using an anti-His antibody (manufactured by Proteintech, 66005-1-Ig).

### Results

The results of the Western blotting are illustrated in FIG. 7. Sample Nos. 2 and 5 correspond to the sample obtained by Culture Method 1; Sample Nos. 1 and 4 correspond to the sample obtained by Culture Method 2; and Sample Nos. 3 and 6 correspond to the sample obtained by Culture Method 3. For Sample Nos. 1, 2, and 3, the electrophoresis was carried out with 10 µL of each sample. For Sample Nos. 4, 5, and 6, the electrophoresis was carried out with 20 µL of each sample. As indicated by the arrows in the figure, bands with the target size were found for Sample Nos. 3 and 6, indicating that the transformed *E. coli* expresses suis PlsA protein.

### Example 7. Production of Plasmalogen in suis plsA-Transformed E. coli

The transformed *E. coli* obtained in Example 6 was subjected to static culture or shake culture, and untransformed *E. coli* (BL21 strain) was used as a subject. According to the procedure described in Example 2, extraction of lipids from bacterial cells, detection of phospholipids, acid hydrolysis treatment, and detection of aldehyde were carried out.

### Results

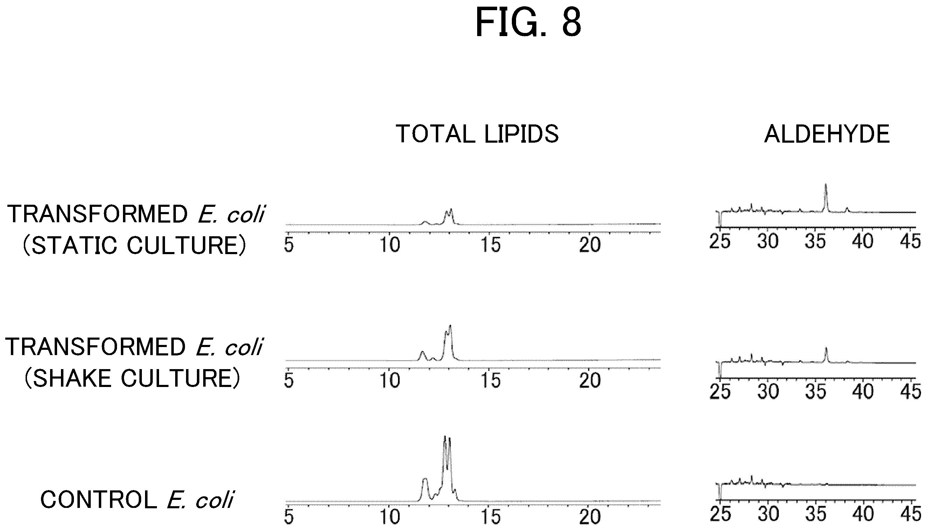

FIG. 8 illustrates chromatograms of the samples detected by ELSD, and the samples treated with the DNPH-HCl solution. In the cases of both the static culture and the shake culture, aldehyde was detected in the transformed *E. coli.* In contrast, no aldehyde was detected in the untransformed *E. coli.* Since acid hydrolysis of plasmalogen generates aldehyde, the transformed *E. coli* was shown to produce plasmalogen. Thus, suis PlsA protein was shown to have a plasmalogen synthesis activity in aerobically grown *E. coli.*

The phospholipid and aldehyde weights per mg wet weight of the *E. coli* bacterial cells as determined from calibration curves are shown in Table 1.

**Table 1**

| | Weight per mg wet weight of bacterial cells | | Aldehyde/Phospholipid (%) |
|---|---|---|---|
| | Phospholipid | Aldehyde | |
| Transformed *E. coli* (Static culture) | 0.90 | 0.04 | 4.5 |
| Transformed *E. coli* (Shake culture) | 1.41 | 0.03 | 2.2 |
| Control *E. coli* | 4.94 | 0.00 | 0.0 |

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-139676, filed on September 2, 2022, the entire disclosure of which is incorporated by reference herein.

## Claims

1. A protein, comprising:
an amino acid sequence selected from a group consisting of following (a), (b), (c), and (d):
(a) an amino acid sequence of SEQ ID NO: 1;
(b) an amino acid sequence having a sequence identity of not less than 80% to the amino acid sequence of SEQ ID NO: 1;
(c) an amino acid sequence of SEQ ID NO:2; and
(d) an amino acid sequence having a sequence identity of not less than 80% to the amino acid sequence of SEQ ID NO:2,
the protein having plasmalogen synthesis activity in a bacterium that expresses the protein.

2. A polynucleotide, comprising:
a base sequence encoding the protein according to claim 1.

3. An expression vector, comprising:
the polynucleotide according to claim 2.

4. A transformant, comprising:
the expression vector according to claim 3.

5. A plasmalogen-containing composition, comprising:
the transformant according to claim 4 or a processed product thereof.

6. A method of producing plasmalogen, the method comprising:
culturing the transformant according to claim 4 under aerobic conditions; and
obtaining plasmalogen from the transformant.
